# EUROPÄISCHE PATENTSCHRIFT

(11) **EP 0 646 107 B1**
(45) Veröffentlichungstag und Bekanntmachung des Hinweises auf die Patenterteilung: **07.10.1998**
(21) Anmeldenummer: 93912920.1
(22) Anmeldetag: 09.06.1993
(51) Int. Cl.: C07C 209/62, C07C 51/06, C07C 51/02, B01D 61/44

(54) **VERFAHREN ZUR GLEICHZEITIGEN HERSTELLUNG VON DICARBONSÄUREN UND DIAMINEN AUS POLYAMIDEN**
METHOD FOR THE SIMULTANEOUS PREPARATION OF DICARBOXYLIC ACIDS AND DIAMINES FROM POLYAMIDES
PROCEDE DE PRODUCTION SIMULTANEE D'ACIDES DICARBOXYLIQUES ET DE DIAMINES A PARTIR DE POLYAMIDES

(30) Priorität: 17.06.1992 DE 4219757
(43) Veröffentlichungstag der Anmeldung: 05.04.1995
(73) Patentinhaber: BASF Aktiengesellschaft, 67063 Ludwigshafen (DE)
(72) Erfinder: SEELIGER, Ursula, D-6700 Ludwigshafen (DE); MUELLER, Wolfgang, F., D-6730 Neustadt (DE); HEIMANN, Frank, D-6700 Ludwigshafen (DE); HUBER, Guenther, D-6701 Dannstadt-Schauernheim (DE); HABERMANN, Wolfgang, D-6500 Mainz 1 (DE); VOSS, Hartwig, D-6710 Frankenthal (DE); SIEGEL, Hardo, D-6720 Speyer (DE)
(86) Internationale Anmeldenummer: EP9301449
(87) Internationale Veröffentlichungsnummer: WO9325514

(56) Entgegenhaltungen:
- WO-A-92/07648
- DE-A- 3 926 642
- DE-B- 1 047 765
- DE-B- 1 163 334
- FR-A- 926 873
- CHEMICAL ABSTRACTS, vol. 53, no. 15, 10. August 1959, Columbus, Ohio, US; abstract no. 14588g, F. CODIGNOLA 'Recovery of primary diamines, especially hexamethylenediamine, from polyamide-resin wastes', & IT,A,533 182 in der Anmeldung erwähnt
- CHEMIE. INGENIEUR. TECHNIK Bd. 57, Nr. 8, 1985, WEINHEIM DE Seiten 702 - 703 H. VOSS 'Einsatz der Elektrodialyse zur Umwandlung organischer Salze in die entsprechenden Säuren'

## Beschreibung

Die vorliegende Erfindung betrifft ein Verfahren zur gleichzeitigen Herstellung von Dicarbonsäuren und Diaminen aus
a) Polymerisaten auf der Basis von Polyamiden aus Dicarbonsäuren oder deren Derivate mit Diaminen, oder
b) Massen, enthaltend im wesentlichen solche Polymerisate,
durch Spaltung dieser Polymerisate in ihre monomeren Bestandteile.

Die Rückspaltung von Polyamiden wie Polyamid 66 ("PA 66") in ihre monomeren Bestandteile kann in neutralem, saurem oder basischem Milieu durchgeführt werden, wobei im allgemeinen die Spaltung in basischem Milieu u.a. wegen der kürzeren Reaktionszeit vorteilhafter ist.

In der FR-A 926 873 wird die Spaltung von Polyamiden wie PA 66 und PA 610 mit anorganischen Basen, beispielsweise mit einer 10 bis 15 gew.-%igen Alkalimetallhydroxidlösung wie Natronlauge, bei 200°C und einem Druck von etwa 15 bar beschrieben. Danach wird das entstandene Diamin durch Extraktion oder Destillation aus dem Reaktionsgemisch abgetrennt und durch Vakuumdestillation weiter gereinigt. Die freie Dicarbonsäure gewinnt man nach dieser Lehre durch Zugabe einer starken Säure wie Salzsäure zur diaminfreien Reaktionsmischung und anschließendes Ausfällen.

In der IT-A 553 182 wird durch einen Überschuß an 20 gew.-%iger Natronlauge bei 220°C und 25 bar eine Verkürzung der Reaktionszeit gegenüber dem Verfahren aus der FR-A 926 873 erreicht. Das Diamin wird mit n-Butanol aus der wäßrigen Lösung extrahiert. Ein Beispiel betrifft die Abtrennung von zuvor im Polymerisat in Form von Fasern enthaltenem unlöslichem Titandioxid durch Filtration nach der Spaltung. Die Freisetzung der Dicarbonsäure erfolgt ebenfalls durch Zugabe einer starken Mineralsäure.

In der FR-A 1 070 841 wird die Spaltung von PA 66 mit Alkalimetall- oder Erdalkalimetallhydroxidlaugen beschrieben. Die Aufarbeitung der Reaktionsmischung erfolgt nach dieser Schrift zunächst durch Ansäuern mit Schwefelsäure und nachfolgender Abtrennung der ausgefallenen Adipinsäure. Anschließend wird das Filtrat mit Kalilauge versetzt, wobei sich Hexamethylendiamin als ölige Schicht abscheidet und abgetrennt und gereinigt werden kann. Daneben wird auch die Spaltung und Aufarbeitung von Polycaprolactam (PA 6) enthaltenden Poly- und Copolymeren beschrieben.

Die DE-A 1 088 063 beschreibt die Spaltung von PA 66 in einer 10 gew.-%igen methanolischen NaOH-Lösung. Das erhaltene Dinatriumadipat wird nach dieser Schrift durch Ansäuern in die freie Säure überführt, Hexamethylendiamin ("HMD") kann durch Destillation in reiner Form gewonnen werden.

Die Spaltung von PA 66 in Dinatriumadipat und HMD in einem Isopropanol- Wasser-Gemisch beschreibt die US-A 2 840 606. Das HMD wird nach dieser Lehre durch Abtrennen aus der Alkoholphase durch Destillation isoliert. Adipinsäure gewinnt man durch Ansäuern der wäßrigen Phase mit Schwefelsäure. Eine Reinigung der Adipinsäure kann durch Kristallisation erfolgen.

Die DE-A 39 26 642 beschreibt ein Verfahren und eine Vorrichtung, basierend auf einer Vierkammerelektrolysezelle, zur Gewinnung einer Säure aus ihrem Salz. Reaktionsparameter und Beispiele werden in der DE-A 39 26 642 jedoch nicht genannt.

Allen diesen Verfahren gemeinsam ist die Gewinnung von Adipinsäure durch Ansäuern der entsprechenden Alkalimetall- oder Erdalkalimetallsalzlösungen. Das hierbei zwangsläufig mitgebildete anorganische Salz, meist Natriumchlorid oder Natriumsulfat, stört einerseits bei der Reinigung der Dicarbonsäure durch Kristallisation, da es diese behindert, und andererseits führen diese Salze zu großen Problemen bei ihrer Entsorgung.

Des weiteren ist von Nachteil, daß mit den beschriebenen Verfahren technisch verwendete, beispielsweise faserverstärkte, mineralgefüllte und/oder schlagzähmodifizierte PA 66 enthaltende Formmassen nicht in geeigneter Weise aufgearbeitet werden können, weil die vielfältigen Zusatzstoffe die Durchführung der beschriebenen Verfahren behindern würden.

Der Erfindung lag daher die Aufgabe zugrunde, ein Verfahren zur gleichzeitigen Herstellung von Dicarbonsäuren und Diaminen zur Verfügung zu stellen, bei dem die oben genannten Nachteile nicht auftreten.

Demgemäß wurde ein Verfahren zur gleichzeitigen Herstellung von Dicarbonsäuren und Diaminen aus
a) Polymerisaten auf der Basis von Polyamiden aus Dicarbonsäuren oder deren Derivate mit Diaminen, oder
b) Massen, enthaltend im wesentlichen solche Polymerisate,
durch Spaltung dieser Polymerisate in ihre monomeren Bestandteile mit einer Base in wäßrigem Milieu und Abtrennung der bei der Spaltung erhaltenen Diamine, wobei man die Base in einer Mischung aus 100 bis 75 Gew.-% Wasser und 0 bis 25 Gew.-% eines C₁-C₄-Alkanols löst und die bei der Spaltung erhaltenen Dicarbonsäuresalze der Adipinsäure oder Sebazinsäure in die entsprechenden Dicarbonsäuren und Basen elektrochemisch überführt, gefunden.

Als Polymerisate auf der Basis von Polyamiden aus Dicarbonsäuren oder deren Derivaten, beispielsweise den entsprechenden Säurehalogeniden, bevorzugt den Säurechloriden, mit Diaminen eignen sich nach den bisherigen Beobachtungen Polyhexamethylenadipinsäureamid, Polyhexamethylensebacinsäureamid und Polytetramethylenadipinsäureamid, bevorzugt Polyhexamethylenadipinsäureamid.

Als Massen, enthaltend im wesentlichen, d.h. zu mindestens 50 Gew.-%, solche Polymerisate, kommen beispielsweise auch Copolyamide mit PA 66 sowie PA 66 oder Copolyamide mit PA 66, enthaltend Fasern und/oder Zusatzstoffe, in Betracht.

Als Basen zur Spaltung der Polymerisate verwendet man in der Regel Alkalimetallhydroxide wie Lithiumhydroxid, Natriumhydroxid und Kaliumhydroxid, bevorzugt Natriumhydroxid, oder deren Mischungen, bevorzugt eine Mischung aus Natrium- und Kaliumhydroxid.

Zweckmäßigerweise setzt man 1,8 bis 4,0, bevorzugt 2,0 bis 3,0, Äquivalente Alkalimetallhydroxid pro wiederkehrende Einheit im Polymeren, beispielsweise -[-(CH₂)₄-CO-NH-(CH₂)₆-NH-CO-]- bei PA 66, ein. Verwendet man weniger als 1,8 Äquivalente der Base, so erhält man in der Regel unerwünschte Anteile an Oligomeren. Bei mehr als 4,0 Äquivalenten der Base pro wiederkehrende Einheit erhält man im allgemeinen, besonders bei glasfaserverstärkten und/oder mineralgefüllten Polyamid-Formmassen, in zu hohem Maße Abbaureaktionen der Glasfasern oder des mineralischen Füllstoffs.

In der Regel setzt man das Alkalimetallhydroxid als 5 bis 25, bevorzugt als 10 bis 15 gew.-%ige Lösung in einer Wasser-C₁-C₄-Alkanol-Mischung ein, die von 0 bis 25 Gew.-%, eines C₁-C₄-Alkanols, oder eine Mischung davon, enthält.

Die Umsetzung nimmt man in der Regel bei einer Temperatur im Bereich von 100 bis 300, bevorzugt von 150 bis 250°C, vor. Der Druckbereich liegt hierbei in der Regel im Bereich von 0,1 bis 10 MPa, wobei man auch außerhalb dieses Druckbereichs arbeiten kann. Bevorzugt arbeitet man unter dem sich bei den Reaktionsbedingungen einstellenden Druck.

Der pH-Wert der Reaktionsmischung liegt im allgemeinen, bedingt durch das Alkalimetallhydroxid, im Basischen, vorzugsweise im Bereich von 8 bis 14.

Die Dauer der Reaktion hängt im wesentlichen von den Konzentrationen der Ausgangsstoffe, der Temperatur und dem Druck ab und liegt in der Regel im Bereich von 0,5 bis 15, vorzugsweise von 3 bis 10 h.

Die Spaltung der erfindungsgemäß eingesetzten Polymerisate bzw. Massen durch eine Base kann man kontinuierlich oder diskontinuierlich durchführen.

Die Spaltung der Polymerisate bzw. Massen durch eine Base kann man in den üblichen Vorrichtungen mit oder ohne Rührer vornehmen, wobei man bevorzugt Druckbehälter mit einem Rührersystem, das besonders gut für Feststoffdispergierung geeignet ist, beispielsweise einem Propellerrührer oder einem Kreuzbalkenrührer, verwendet.

In einer bevorzugten Ausführungsform zerkleinert man das eingesetzte Polymerisat bzw. polyamidhaltige Massen mechanisch auf eine mittlere Teilchengröße von 0,1 bis 50, bevorzugt von 1 bis 10 mm, bevor man es bzw. sie der Spaltung zuführt. Die Zerkleinerung kann man in einer handelsüblichen Mühle, beispielsweise in einer Schneidmühle, oder, bevorzugt, insbesondere wenn die eingesetzten Massen harte Materialien wie Metalleinlegeteile, z.B. Schrauben, enthalten, in einer Hammermühle.

Aus dem auf diese Weise zerkleinerten Material kann man vorhandene Metallteile in einem Trockentrennverfahren mit einem sogenannten Luftherd, bevorzugt mit anschließender Induktionsabscheidung, z.B. mit Hilfe eines Freifall-Rohrabscheiders, zur vollständigen Abtrennung der Metallteile, oder in einem Naßtrennverfahren, beispielsweise mittels eines Hydrozyklons, entfernen.

In einer besonders bevorzugten Ausführungsform zerkleinert man die einzusetzenden Polymerisate bzw. Massen auf eine Größe von maximal 50 mm Länge in einer Hammermühle, trennt gewünschtenfalls vorhandene Metallteile ab, und zerkleinert anschließend das von Metallteilen befreite Mahlgut auf eine Größe im Bereich von 5 bis 12 mm auf einer Schneidmühle. Gegebenenfalls kann man daran anschließend das so aufbereitete Polymerisat bzw. polymerisathaltige Masse noch waschen und trocknen, bevor man es bzw. sie der Spaltung durch eine Base zuführt.

Die nach der Spaltung der Polyamide erhaltene Reaktionsmischung besteht erfindungsgemäß aus einer flüssigen Phase, enthaltend das Diamin und das Dicarbonsäuresalz, sowie unlöslichen Bestandteilen.

Die nach der Spaltung der Polyamide erhaltene Reaktionsmischung unterzieht man erfindungsgemäß anschließend einer elektrochemischen Behandlung zur Überführung des Dicarbonsäuresalzes in die entsprechende Dicarbonsäure, wobei es vorteilhaft ist, zuvor störende Verunreinigungen abzutrennen.

So entfernt man in einer weiteren bevorzugten Ausführungsform nach der Spaltung der Polyamide in ihre monomeren Bestandteile unlösliche Bestandteile aus der Reaktionsmischung.

Als unlösliche Bestandteile seien beispielhaft genannt Glasfasern, Kohlenstoffasern, Ruß, Mineralien und Kautschuk sowie zuvor nicht oder nicht vollständig abgetrennte Metalle soweit sie nicht durch die Base in Lösung gebracht wurden.

Als Verfahren zum Abtrennen unlöslicher Bestandteile eignen sich bekannte Verfahren wie Filtration, Sedimentation oder Zentrifugieren.

Die abgetrennten, unlöslichen Bestandteile kann man gewünschtenfalls in einem weiteren Verfahrensschritt mit Wasser und/oder einem organischen Lösungsmittel, bevorzugt ein C₁-C₄-Alkohol oder der bei der Basenspaltung verwendete Lösungsmittelkomponente, waschen. Diesen Verfahrensschritt kann man mit dem für die Abtrennung eingesetzten Apparat, beispielsweise einem Bandfilter, und/oder bei einer weiteren Trennoperation durchführen, wobei die unlöslichen Bestandteile zuvor in der Regel mit dem verwendeten Wasser oder Lösungsmittel(gemisch) innig gemischt werden. Die hierbei erhaltenen Feststoffe kann man gegebenenfalls nach dem Trocknen als Füllstoff weiterverwenden.

Die Filtrate aus den Waschoperationen kann man gewünschtenfalls mit dem Filtrat der Reaktionsmischung vereinigen.

Eine weitere bevorzugte Ausführungsform besteht darin, daß man vorzugsweise nach der Abtrennung unlöslicher Bestandteile und vor der elektrochemischen Behandlung die bei der Basenspaltung erhaltenen Diamine abtrennt.

Die Abtrennung der Diamine kann man nach bekannten Verfahren wie Destillation, insbesondere Rektifikation, oder Extraktion durchführen, wobei aus energetischen Gründen die Extraktion mit organischen Lösungsmitteln bevorzugt ist.

Vor der Extraktion kann es zweckmäßig sein, leichtflüchtige organische Komponenten, soweit diese vorhanden sind, beispielsweise durch Rektifikation abzutrennen. Zur Rektifikation können an sich bekannte Apparaturen (z.B. Bodenkolonnen oder Kolonnen mit geordneten oder ungeordneten Packungen) eingesetzt werden.

Zur Extraktion der Diamine kann man die üblichen bekannten Lösungsmittel (s. DE-A 1,163,334) wie halogenierte Kohlenwasserstoffe, beispielsweise Chloroform oder Methylenchlorid, C₄-C₈-Alkohole wie n-Butanol, i-Butanol, sek.-Butanol, 1-Pentanol, 2-Pentanol, 3-Pentanol, Neopentylalkohol, 1-Hexanol, 2-Hexanol, 3-Hexanol, 1-Heptanol, 2-Heptanol, 3-Heptanol, 4-Heptanol, 1-Octanol, 2-Octanol, 3-Octanol, 4-Octanol, bevorzugt n-Butanol, i-Butanol, C₅-C₈-Cycloalkane wie Cyclopentan, Cyclohexan, Cycloheptan, Cyclooctan, bevorzugt Cyclohexan, sowie aromatische Kohlenwasserstoffe, bevorzugt Benzol und dessen C₁-C₄-Alkylderivate wie Toluol und Xylole, sowie Mischungen dieser Lösungsmittel verwenden. Besonders bevorzugt setzt man Mischungen von Benzol, Toluol, n- und i-Butanol ein. Ganz besonders bevorzugt sind Mischungen aus von 25 bis 60 Gew.-% Benzol und/oder Toluol und von 40 bis 75 Gew.-% einer Mischung aus n- und i-Butanol, wobei man den Gesamtgehalt an i-Butanol in der Mischung in der Regel nicht höher als 40 Gew.-% wählt.

Die Extraktion kann man diskontinuierlich oder kontinuierlich im allgemeinen in an sich bekannten Extraktionsapparaturen wie in Mixer-Settlern oder in gepulsten oder ungepulsten Extraktionskolonnen (z.B. in Siebboden- oder Füllkörperkolonnen) durchführen.

Die Isolation der Diamine aus dem Extrakt erfolgt in der Regel durch eine Rektifikation in an sich bekannten Apparaturen, wobei man die Diamine in einer vorteilhaften Ausführung als dampfförmigen Seitenabzug im Abtriebsteil der Rektifikationskolonne erhält. Die Rektifikation erfolgt im allgemeinen bei einem Druck im Bereich von 10 bis 100 kPa, bevorzugt von 50 bis 80 kPa.

Aus dem Rektifikationsrückstand kann man gewünschtenfalls noch vorhandene Reste an Diamin in einem weiteren Destillationsschritt, beispielsweise mit einem Dünnschichtverdampfer, bei einem Druck im Bereich von 0,5 bis 50, bevorzugt von 2 bis 30 kPa, abtrennen.

Das am Kopf der Rektifikationskolonne erhaltene Extraktionsmittel führt man zweckmäßig direkt in die Extraktion zurück oder befreit es in einem weiteren Destillationsschritt von leichter- und/oder schwerersiedenden Verunreinigungen.

Das Raffinat kann man gewünschtenfalls einer weiteren Rektifikation unterziehen, um gelöstes Extraktionsmittel und/oder andere flüchtige organische Verbindungen abzutrennen.

Die elektrochemische Behandlung störenden Verunreinigungen - wie Erdalkalimetallkationen, Kieselsäure-, Polyphosphatanionen oder hochmolekulare organische Aminverbindungen - kann man zweckmäßig aus den von unlöslichen Bestandteilen und Diaminen befreiten wäßrigen Lösungen entfernen, indem man diese Lösungen beispielsweise mit Adsorptionsmitteln und/oder geeigneten Fällungsmitteln behandelt.

Als Adsorptionsmittel setzt man bevorzugt Aktivkohle oder selektive Ionenaustauscher, gewünschtenfalls auf Basis von Chelaten, ein. Als Fällungsmittel kann man beispielsweise Carbonate der Alkalimetalle und/oder Ammoniumcarbonate einsetzen.

Nach den bisherigen Beobachtungen hat die Art der elektrochemischen Behandlung keinen prinzipiellen Einfluß auf das erfindungsgemäße Verfahren.

Beispielhaft genannt seien die folgenden Verfahrensvarianten (a) bis (f):
(a) Bei dieser Verfahrensweise kann man die Spaltung des Dicarbonsäuresalzes in die entsprechende Dicarbonsäure und die entsprechende Base in einer zweigeteilten Elektrodialysezelle mit bipolaren Membranen durchführen. Im allgemeinen ist die Elektrodialysezelle so aufgebaut, daß zwischen Anode und Katode 1 bis 200, bevorzugt 20 bis 70 Elektrodialyseeinheiten zusammengefaßt werden, die voneinander durch bipolare Membranen getrennt sind. Die bipolaren Membranen trennt man voneinander durch Kationenaustauschermembranen ab, wodurch der folgende Aufbau in einer Elektrodialyseeinheit vorliegt: bipolare Membran (anodenseitig) - Anolytkammer - Kationenaustauschermembran - Katolytkammer - bipolare Membran (katodenseitig). Die einzelnen Elektrodialyseeinheiten schaltet man zweckmäßig elektrisch in Reihe.
   Bei dieser Verfahrensweise führt man zweckmäßigerweise die wäßrige Dicarbonsäuresalzlösung der Anolytkammer zu. Im elektrischen Feld einer angelegten Gleichspannung wandern im allgemeinen die Alkalimetallkationen durch die Kationenaustauschermembran in die Katolytkammer. Die zur Kompensation der getrennten Ladungen erforderlichen Hydroxyl-Anionen, die durch Dissoziation des Wassers in den bipolaren Membranen gebildet werden, stammen aus der katodenseitigen bipolaren Membran. Auf diese Weise reichert sich in der Katolytkammer die entsprechende Alkalimetallhydroxid-Lösung an. In der Anolytkammer kann das Dicarbonsäureanion mit den Wasserstoffionen, die aus der anodenseitigen bipolaren Membran stammen, die freie Dicarbonsäure bilden.
   Vorteilhaft führt man die Dicarbonsäuresalzlösung parallel den Anolytkammern zu. Die Produktströme aus den Anolytkammern, enthaltend die freie Säure und nicht umgesetztes Dicarbonsäuresalz, sowie die Produktströme der Katolytkammern faßt man zweckmäßig jeweils zusammen. Die freie Dicarbonsäure gewinnt man in der Regel durch Auskristallisieren aus den zusammengeführten Produktströmen der Anolytkammer, ohne daß das entsprechende Dicarbonsäuresalz, das man vorzugsweise erneut der Elektrodialyse unterwirft, mitausfällt.
   Die Elektrodialyse kann man sowohl kontinuierlich als auch diskontinuierlich durchführen. Eine bevorzugte Ausführungsform bei der kontinuierlichen Verfahrensweise mit mehreren Elektrodialysezellen besteht darin, daß man den Gesamtumsatz auf 2 bis 20, bevorzugt 4 bis 6 Elektrodialysezellen verteilt, und pro Elektrodialysezelle nur einen Teilumsatz vornimmt.
   Besonders vorteilhaft führt man dabei die Stoffströme nach dem Gegenstromprinzip. Der Ablauf der Anolytkammer bildet den Zulauf der darauffolgenden Anolytkammer usw., so daß der Ablauf der letzten Anolytkammer mit Dicarbonsäure angereichert und mit Dicarbonsäuresalz abgereichert ist. Der Ablauf der letzten Katolytkammer, enthaltend eine niedrige Konzentration an Alkalimetallhydroxid, bildet den Zulauf der vorletzten Katolytkammer usw., so daß in der ersten Einheit eine hohe Konzentration an Dicarbonsäuresalz in der Anolytkammer und eine hohe Alkalimetallhydroxidkonzentration in der Katolytkammer vorliegen. Damit erreicht man, daß die Konzentrationsunterschiede an Alkalimetallhydroxid in den Anolyt- und Katolytkammern pro Einheit klein sind. Letztlich führt dies in der Regel zu einer Energieeinsparung durch eine höhere Stromausbeute und im Mittel zu niedrigeren Zellspannungen.
   Die Stromdichten liegen im allgemeinen im Bereich von 0,1 bis 2, bevorzugt von 0,5 bis 1,0 kA/m². Die Zellspannung beträgt in der Regel pro Elektrodialyseeinheit 3 bis 8 V, bevorzugt 4 bis 6 V.
   Die pH-Werte liegen im allgemeinen in den Anolytkammern im Bereich von 2 bis 10, in den Katolytkammern im Bereich größer als 13.
   Die Kammerbreite beträgt in der Regel 0,2 bis 5, bevorzugt 0,5 bis 1 mm.
   Die Temperatur während der Elektrodialyse wählt man im allgemeinen im Bereich von 40 bis 110, vorzugsweise von 65 bis 90°C.
   Die Strömungsgeschwindigkeiten der Zu- und Abläufe liegt im allgemeinen im Bereich von 0,05 bis 0,2 m/sec.
   Die Konzentration an eingesetztem Dicarbonsäuresalz beträgt in der Regel 5 bis 40 Gew.-%, bevorzugt 10 bis 20 Gew.-%.
   Gewünschtenfalls kann man die Leitfähigkeit im Anolytsystem durch Zusätze von Salzen oder Säuren wie Natriumsulfat oder Schwefelsäure erhöhen. Solche Stoffe setzt man im allgemeinen im Bereich von 0,1 bis 10 Gew.-%, bevorzugt von 1 bis 6 Gew.-%, bezogen auf das Gesamtgewicht der in der Anolytkammer befindlichen Lösung, ein.
   Der Katolytkammer kann man zweckmäßig die Stoffe, die im Betrieb erhalten werden, vorzugsweise das entsprechende Alkalimetallhydroxid wie Natrium- und Kaliumhydroxid, vorzugsweise Natriumhydroxid, zusetzen.
   Als Zulauf in die Katolytkammer verwendet man in der Regel vollentsalztes Wasser ("VE-Wasser"), wobei man vorzugsweise zu Beginn 1 bis 25, bevorzugt 5 bis 10 gew.-%ige, bei der Elektrodialyse entstehende Alkalimetallhydroxidlösung einsetzt.
b) Eine dreigeteilte Elektrodialysezelle mit bipolaren Membranen hat den Vorteil gegenüber der unter (a) aufgeführten Verfahrensweise, daß keine hohen Anforderungen an die Reinheit der Einsatzstoffe gestellt zu werden brauchen. Des weiteren erhält man im allgemeinen wesentlich geringere Salzanteile sowohl in der erhaltenen Dicarbonsäurelösung als auch in der entsprechenden Alkalimetallhydroxidlösung.
   Das Dreikammersystem enthält neben einer Kationenaustauschermembran eine Anionenaustauschermembran, so daß sich für eine Elektrodialyseeinheit folgender Aufbau ergibt: bipolare Membran (anodenseitig) - Anolytkammer - Anionenaustauschermembran - Mittelkammer - Kationenaustauschermembran - Katolytkammer - bipolare Membran (katodenseitig).
   Die Dicarbonsäuresalzlösung führt man zweckmäßig in die Mittelkammer zu. In einem durch Gleichspannung angelegten elektrischen Feld wandern in der Regel die Dicarbonsäureanionen durch die Anionenaustauschermembran in die Anolytkammer, wo sie mit den dort vorhandenen Wasserstoffionen die freie Säure bilden können. Abgesehen von Selektivitätsverlusten der Anionenaustauschermembran kann man der Anolytkammer die freie Säure salzfrei entnehmen. In der Katolytkammer fällt wie bei (a) die Alkalihydroxidlösung an. Den Ablauf der Mittelkammer, enthaltend noch Restmengen an Dicarbonsäuresalz kann man entsorgen oder zweckmäßig erneut dem Zulaufstrom zur Elektrodialyse zuführen. Analog zu (a) kann man die Stoffströme im Gegenstromprinzip führen, um die Stromausbeute zu erhöhen.
   Zur Erhöhung der Leitfähigkeit kann man der Anolytkammer beispielsweise eine Oxosäure wie Schwefelsäure, Phosphorsäure und Salpetersäure zusetzen.
   Der Katolytkammer kann man zweckmäßig die Stoffe, die im Betrieb erhalten werden, vorzugsweise das entsprechende Alkalimetallhydroxid wie Natrium- und Kaliumhydroxid, vorzugsweise Natriumhydroxid, zusetzen.
   Im übrigen kann man das Verfahren nach (b) unter den gleichen Bedingungen wie unter (a) beschrieben durchführen.
(c) Prinzipiell kann man auch Elektrodialysezellen mit vier Kammern verwenden. Der Aufbau gleicht im allgemeinen dem einer Elektrodialysezelle mit drei Kammern, mit dem Unterschied, daß zum Schutz der bipolaren Membranen vor möglichen störenden Verunreinigungen eine weitere Ionenaustauschermembran, vorzugsweise eine Kationenaustauschermembran, eingebaut ist. Es liegt in der Regel folgender Aufbau in einer Elektrodialyseeinheit vor:
   bipolare Membran (anodenseitig) - Anolytkammer - Kationenaustauschermembran - anodennahe Mittelkammer - Anionenaustauschermembran - katodennahe Mittelkammer - Kationenaustauschermembran - Katolytkammer - bipolare Membran (katodenseitig).
   Zweckmäßig führt man die Dicarbonsäurelösung der katodennahen Mittelkammer zu, und führt aus der anodennahen Mittelkammer die Dicarbonsäurelösung und aus der Katodenkammer die Alkalimetallhydroxidlösung ab.
   Im übrigen kann man das Verfahren nach (c) unter den gleichen Bedingungen wie unter (b) beschrieben durchführen.
(d) Die elektrochemische Spaltung der Dicarbonsäuresalze in Dicarbonsäure und die betreffende Base kann man nach einer weiteren Ausführungsform in einer an sich aus der Chlor-Alkalielektrolyse bekannten zweigeteilten membranelektrolysezelle durchführen. Die Membranelektrolysezelle ist im allgemeinen so aufgebaut, daß 1 bis 100, bevorzugt 20 bis 70 Elektrolyseeinheiten zu einem Zellblock zusammengefaßt werden. Dabei können die einzelnen Elektrolyseeinheiten elektrisch in Reihe geschaltet werden, indem man die Katode einer Einheit mit der Anode der nächsten Einheit elektrisch leitend verbindet, oder intern verbundene bipolare Elektroden einsetzt. Die Produktzufuhr und die Produktabfuhr erfolgt in der Regel über Sammelleitungen für jeden Kammertyp getrennt. Die zweigeteilte Membranelektrolyseeinheit ist im allgemeinen in Richtung von Anode zur Katode wie folgt aufgebaut:
   Anode-Anolytkammer-Kationenaustauschermembran-Katolytkammer-Kathode.
   Die wäßrige Dicarbonsäuresalzlösung wird zweckmäßig der Anolytkammer zugeführt. Im elektrischen Feld der angelegten Gleichspannung werden im allgemeinen die Alkalimetallkationen durch die Kationenaustauschermembran in die Katolytkammer überführt und dort zu Lauge umgesetzt. Die zur Kompensation der getrennten Ladungen erforderlichen Hydroxylanionen werden bei der Katodenreaktion freigesetzt. Die Katodenreaktion kann zum Beispiel die katodische Wasserstoffabscheidung oder zum Beispiel eine katodische Reduktion von Sauerstoff sein. In der Anolytkammer verbleibt im allgemeinen der organische Säurerest, der mit den Wasserstoffionen bzw. deren hydratisierte Formen, die bei der Anodenreaktion frei werden, die entsprechende freie Säure bildet. Als Anodenreaktion sei zum Beispiel die anodische Sauerstoffabscheidung oder die anodische Oxidation von Wasserstoff genannt. In der Anodenkammer reichert sich somit in der Regel das Salz ab und der Gehalt an freier Dicarbonsäure nimmt zu.
   Die Membranelektrolyse kann sowohl diskontinuierlich, als auch kontinuierlich durchgeführt werden. Bei der kontinuierlichen Fahrweise bietet es sich an, den Umsatz auf 2 bis 20, bevorzugt 4 bis 6 Zellen zu verteilen und die Stoffströme nach dem Gegenstromprinzip zu führen (s. (a)).
   Die eingesetzte Dicarbonsäuresalzlösung, gegebenenfalls enthaltend mehrere solcher Salze, setzt man in der Regel in einer Konzentration von 1 Gew.-% bis zur Sättigungskonzentration des oder der entsprechenden Salze, bevorzugt von 5 bis 35, besonders bevorzugt von 15 bis 30 Gew.-%, ein.
   Die Stromdichten liegen im allgemeinen im Bereich von 0,5 bis 10, bevorzugt von 1 bis 4 kA/m². Die Zellspannung beträgt in der Regel pro Membranelektrolyseeinheit 3 bis 10 V, bevorzugt 4 bis 6 V.
   Die pH-Werte liegen im allgemeinen in den Anolytkammern im Bereich von 2 bis 10, in den Katolytkammern im Bereich größer als 13.
   Die Kammerbreite beträgt in der Regel 0,5 bis 10, bevorzugt 1 bis 5 mm.
   Die Temperatur während der Membranelektrolyse wählt man im allgemeinen im Bereich von 50 bis 110, vorzugsweise von 65 bis 90°C.
   Um den Stofftransport zu gewährleisten, wälzt man im allgemeinen die Kammerinhalte entweder mittels Pumpen oder im Naturumlauf, d.h. durch Mammutpumpeneffekt der Gasentwicklung an den Elektroden, um. Die Strömungsgeschwindigkeiten in den Kammern liegen im allgemeinen im Bereich von 0,05 bis 0,5, bevorzugt von 0,1 bis 0,2 m/sec.
(e) Eine besonders bevorzugte Ausführungsform ist die elektrochemische Spaltung der Dicarbonsäuresalze in Dicarbonsäure und die betreffende Base in einer dreigeteilten Membranelektrolysezelle.
   Die dreigeteilte Membranelektrolyseeinheit ist in der Regel wie folgt aufgebaut:
   Anode-Anolytkanmer-Kationenaustauschermembran-Mittelkanmer-Kationenaustauschermembran-Katolytkammer-Katode.
   Die wäßrige Dicarbonsäuresalzlösung wird im allgemeinen der Mittelkammer zugeführt. Um die elektrische Leitfähigkeit in der Mittelkammer zu erhöhen, kann man eine Mineralsäure oder ein Salz dem Mittelkammerelektrolyten beigeben. Beispielhaft genannt seien Schwefelsäure, Salpetersäure, Natriumsulfat und Natriumnitrat.
   In der Mittelkammer verbleibt im allgemeinen der organische Säurerest, der mit den Wasserstoffionen, die bei der Anodenreaktion frei werden und über die anodenseitige Kationenaustauschermembran in die Mittelkammer überführt werden, zu freier Säure reagieren kann. Die Säure wird in der Regel zusammen mit dem nicht umgesetzten Salz dem Mittelkammersystem entnommen. Als Anolyt kann man eine wäßrige Mineralsäure wie Schwefelsäure, Salpetersäure oder Salzsäure einsetzen, bevorzugt Schwefelsäure. Der Anolyt dient im wesentlichen zusammen mit der anodenseitigen Kationenaustauschermembran zum Schutz der organischen Dicarbonsäure vor anodischer Oxidation.
   Im übrigen kann man das Verfahren nach (e) unter den bei (d) genannten Bedingungen durchführen.
(f) Die elektrochemische Spaltung der Dicarbonsäuresalze in Dicarbonsäure und die betreffende Base kann man auch in einer viergeteilten Membranelektrolysezelle durchführen.
   Die viergeteilte Membranelektrolyseeinheit ist im allgemeinen wie folgt aufgebaut:
   Anode-Anolytkammer-Kationenaustauschermembran-anodennahe Mittelkammer-Anionenaustauschermembran-katodennahe Mittelkammer-Kationenaustauschermembran-Katolytkammer-Katode.
   Die wäßrige Dicarbonsäuresalzlösung führt man zweckmäßig der katodennahen Mittelkammer zu.
   Um die elektrische Leitfähigkeit in der Mittelkammer zu erhöhen, kann man eine Mineralsäure oder ein Salz wie Schwefelsäure, Salpetersäure, Natriumsulfat und Natriumnitrat dem Mittelkammerelektrolyten beigeben.
   Aus der katodennahen Mittelkammer wird im allgemeinen das Säureanion in die anodennahe Mittelkammer überführt, wo es mit Wasserstoffionen, die bei der Anodenreaktion frei werden und über die anodenseitige Kationenaustauschermembran in die anodennahe Mittelkammer gelangen, zu freier Säure reagiert. Die Säure wird in der Regel mit hoher Reinheit dem Mittelkammersystem entnommen. Die Restsalzlösung wird im allgemeinen der katodennahen Mittelkammer entnommen und im Teilstrom in die Adipatlösestufe zurückgeführt oder entsorgt. Als Anolyten setzt man in der Regel eine wäßrige Mineralsäure ein, bevorzugt Schwefelsäure. Der Anolyt dient im wesentlichen zusammen mit der anodenseitigen Kationenaustauschermembran zum Schutz der organischen Säure vor anodischer Oxidation.
   Im übrigen kann man das Verfahren nach (f) unter den bis (d) genannten Bedingungen durchführen.

Bei den vorgenannt beschriebenen Verfahrensvarianten verwendet man als Kationenaustauschermembranen besonders bevorzugt Polymere auf Basis von perfluorierten Olefinen oder Copolymere aus Styrol und Divinylbenzol, die als ladungstragende Gruppen Sulfonsäure- und gewünschtenfalls Carboxylgruppen enthalten. Ganz besonders bevorzugt verwendet man Membranen, die nur Sulfonsäuregruppen enthalten, da sie in der Regel beständiger gegenüber Verschmutzungen durch mehrwertige Kationen sind als andere Membranen. Solche Membranen sind bekannt (beispielsweise sogenannte Nafionmembranen vom Typ 324). Sie bestehen aus einem Copolymer von Tetrafluorethylen und einem perfluorierten Monomer, das Sulfongruppen enthält. Sie weisen in der Regel eine hohe chemische und thermische Stabilität auf. Zur mechanischen Verstärkung kann die Ionenaustauschermembran durch ein Teflonstützgewebe stabilisiert sein. Weiterhin sind Copolymere auf der Basis von Styrol und Divinylbenzol geeignet.

Als Anionenaustauschermembranen kann man beispielsweise die in der EP-A 449 071 ausführlich beschriebenen Membranen verwenden, so daß sich weitere Ausführungen hierzu erübrigen.

Als Elektrodenmaterialien kann man im allgemeinen perforierte Materialien verwenden, die z.B. in Form von Netzen, Lamellen, Ovalprofilstegen oder Rundprofilstegen ausgeführt sind.

Die Sauerstoffüberspannung der Anoden wählt man in der Regel im erfindungsgemäßen Stromdichtebereich weniger als 400 mV, damit es nicht zur Bildung von Ozon oder Perverbindungen kommt.

Geeignete Anodenmaterialien mit geringen Sauerstoffüberspannungen sind beispielsweise Titanträger mit elektrisch leitenden Zwischenschichten aus Boriden und/oder Carbiden und/oder Siliciden der IV. bis VI. Nebengruppe wie Tantalboride, Titanboride oder Titansuboxid sowie gegebenenfalls dotierte Zinnoxide oder gewünschtenfalls mit Platinmetallen dotiertes Tantal und/oder Niob, deren Oberfläche im allgemeinen mit elektrisch leitenden, nicht stöchiometrischen Mischoxiden der IV. bis VI. Nebengruppe und Metallen oder Metalloxiden der Platingruppe oder Platinmetallverbindungen wie Platinaten dotiert sind. Auf diesen Zwischenschichten befindet sich im allgemeinen das aktive Elektrodenmaterial, das bevorzugt aus Mischoxiden des Tantals mit Iridium, Platin oder Rhodium sowie Platinaten vom Typ Li_{0,3}Pt₃O₄ besteht. Zur Vergrößerung der Oberfläche verwendet man in der Regel oberflächlich aufgerauhte oder makroporöse Titanträger.

Als Katoden setzt man im allgemeinen Elektrodenmaterialien mit einer niedrigen Wasserstoffüberspannung ein, um zusätzliche Spannungsverluste in der Membranelektrolyse- oder Elektrodialysezelle zu vermeiden. Geeignete Katoden sind beispielsweise Eisen- oder Nickelträger, die oberflächlich mit feinteiligem Kobalt, Nickel, Molybdän, Wolfram, Mangan, Raneymetallverbindungen des Nickels oder Kobalts, Nickel- oder Kobalt-Aluminiumlegierungen, oder Nikkel-Eisenlegierungen oder Kobalt-Eisenlegierungen mit 65 bis 90 Gew.-% Eisen beschichtet sind.

Zur Verbesserung der Selektivität und der Membranstandzeiten können auf der Kationenseite Kationenaustauschermembranen mit Hydroxylionenblockern verwendet werden. Man kann die Selektivität ferner dadurch verbessern, daß man den Gehalt an Calcium-, Magnesium- und Aluminiumionen sowie den Kieselsäureanteil kleiner als jeweils 5 ppm hält.

Die nach der elektrochemischen Behandlung gewonnene Dicarbonsäure liegt im allgemeinen als wäßrige Lösung in einer Konzentration im Bereich von 1 bis 30, bevorzugt von 4 bis 30 Gew.-%, vor. Diese Lösung kann gegebenenfalls vorhandenes Leitfähigkeitssalz in einer Konzentration im Bereich von 0,05 bis 15, bevorzugt von 0,06 bis 6 Gew.-%, und gegebenenfalls vorhandene Mineralsäure in einer Konzentration im Bereich von 0,05 bis 15, bevorzugt von 0 bis 6 Gew.-%, enthalten.

Die erfindungsgemäß erhaltene Lauge enthält in der Regel ein Alkalimetallhydroxid in einer Konzentration im Bereich von 5 bis 35, vorzugsweise von 15 bis 25 Gew.-%.

Besonders bevorzugt kann man die erfindungsgemäß erhaltene Alkalimetallhydroxidlösung in den Kreis..auf zurückführen oder anderweitig verwenden, wobei man sie gewunschtenfalls zuvor in an sich bekannter Weise, beispielsweise durch Eindampfen, aufkonzentrieren kann.

Um die Dicarbonsäure in reiner Form zu erhalten, kristallisiert man sie hierzu in der Regel aus der erfindungsgemäß erhaltenen Lösung aus, trennt das Produkt anschließend, beispielsweise durch Filtration, ab und trocknet es.

Vorzugsweise erhält man die Dicarbonsäure aus den bei der Elektrodialyse bzw. Membranelektrolyse erhaltenen Lösungen, durch Kühlungs- oder Verdampfungskristallisation. Anschließend trennt man im allgemeinen die Dicarbonsäuren aus den so erhaltenen Suspensionen ab, z.B. durch Filtration, Dekantieren oder Zentrifugieren.

Die Kühlungskristallisation führt man üblicherweise bei einer Temperatur im Bereich von 0 bis 50°C, bevorzugt von 10 bis 40°C, wobei man zweckmäßig bei Drücken im Bereich von 1 bis 100 kPa, vorzugsweise von 4 bis 20 kPa arbeitet, durch.

Die abgetrennten Dicarbonsäuren kann man vorzugsweise durch Waschen, beispielsweise mit Wasser oder C₁-C₄-Alkanolen, und gewünschtenfalls durch Umkristallisieren in reiner Form erhalten. Bei gleichzeitigem Vorliegen von mehreren Dicarbonsäuren kann man aufgrund der unterschiedlichen Löslichkeiten nach bekannten Methoden wie fraktioniertes Kristallisieren die einzelnen Dicarbonsäuren in reiner Form isolieren.

Die bei der Kristallisation und dem Waschen anfallenden wäßrigen Lösungen kann man wie üblich aufkonzentrieren und erneut der Kristallisation unterwerfen, beispielsweise dadurch, daß man sie zu noch zu kristallisierenden Lösungen, die direkt nach der Elektrodialyse bzw. Membranelektrolyse anfallen, hinzugibt. Man kann sie beispielsweise auch der Reaktionsmischung, die man nach der Behandlung der eingesetzten Polymerisate bzw. Massen mit einer Base erhält, zuführen.

Ein Vorteil des erfindungsgemäßen Verfahrens gegenüber bekannten Verfahren liegt darin, daß die Bildung und Entsorgung von Salzen, die üblicherweise bei der Freisetzung der Dicarbonsäuren aus ihren Salzen durch Ansäuern anfallen, entfällt. Ein weiterer Vorteil besteht darin, daß auch faserverstärkte, mineralgefüllte und/oder schlagzähmodifizierte Formmassen aufgearbeitet werden können. Des weiteren kann man die durch das erfindungsgemäße Verfahren erhaltenen Stoffe wie Dicarbonsäuren, Diamine und Basen sowie gegebenenfalls Glasfasern und mineralische Füllstoffe zur Herstellung neuer Produkte verwenden.

### Beispiel 1

300 g eines auf ca. 8 mm (mittlerer Teilchendurchmesser) zerkleinertes Polyamid 66 mit einer Viskositätszahl (VZ) = 149 (Einheit: 1 cm³/g (gemessen an einer 0,5 gew.-%igen Lösung des Polyamids in 96 gew.-%iger Schwefelsäure bei 25°C nach DIN 53 727) wurden in einem Druckbehälter unter Rühren mit 780 g einer 15gew.-%igen wäßrigen Natriumhydroxidlösung für 6 Stunden auf 220°C erhitzt.

Nach dem Abkühlen wurde eine leicht gelblich gefärbte, homogene wäßrige Lösung erhalten, die die Reaktionsprodukte Hexamethylendiamin und Natriumadipat enthielt.

Zur Abtrennung des Hexamethylendiamins wurde die Lösung mehrmals mit insgesamt 800 g einer Mischung aus 50 Vol.-% Toluol und 50 Vol.-% n-Butanol extrahiert. Die vereinigten organischen Phasen wurden einer fraktionierten Destillation unterworfen. Unter Normaldruck wurden zunächst Toluol, n-Butanol und Wasser abgetrennt.

Bei 128 bis 131°C/100 mbar wurden 141 g Hexamethylendiamin als farblose Schmelze erhalten.

Die nach der Extraktion verbleibende wäßrige Natriumadipatlösung wurde unter Normaldruck bis zu einer 27 gew.-%igen Natriumadipatlösung eingedampft, wobei Reste des Extraktionsmittels mit entfernt wurden. Die konzentrierte Natriumadipatlösung wurde anschließend mit 0,5 g gepulverter Aktivkohle pro 100 ml Lösung versetzt und auf 50°C erwärmt. Nach 1 h wurde die Aktivkohle abfiltriert und unter Rühren wurden 80 mg Natriumcarbonat pro 100 g Lösung zugesetzt. Nach 1 h wurde der Rührer abgeschaltet und nach weiteren 4 h wurde die Lösung filtriert. Diese vorgereinigte Natriumadipatlösung wurde dann einer Behandlung mit einem Selektiv-Ionenaustauscherharz (Lewatit TP 208 (von BAYER)) unterworfen.

### Beispiel 2

Für diesen Versuch wurde ein auf ca. 8 mm (mittlerer Teilchendurchmesser) zerkleinertes, (mit Ruß) schwarz eingefärbtes, (wärmestabilisiertes) und glasfaserverstärktes Polyamid 66 mit einer Viskositätszahl (VZ) = 140 (gemessen nach DIN 53 727), s. Beispiel 1) und einem Glasfaseranteil von 36 Gew.-% (Bestimmung des Glühverlustes von glasfaserverstärkten Kunststoffen nach DIN 53 395) verwendet. In einem Druckbehälter wurden unter Rühren 490 g dieses Verbundmaterials mit 1180 g einer 10 gew.-%igen Natriumhydroxidlösung für 8 Stunden auf 220°C erhitzt. Nach dem Abkühlen der Reaktionsmischung wurden die unlöslichen Bestandteile wie Glasfasern abfiltriert und mehrmals mit Wasser gewaschen.

Das Mutterfiltrat und die vereinigten Waschfiltrate wurden zur Abtrennung des Hexamethylendiamins mehrmals mit insgesamt 1100 g einer Mischung aus 50 Vol.-% Toluol und 50 Vol.-% n-Butanol extrahiert. Die vereinigten organischen Phasen wurden einer fraktionierten Destillation unterworfen. Zunächst wurden unter Normaldruck Leichtsieder wie Toluol, n-Butanol und Wasser abgetrennt. Bei 128 bis 131°C/100 mbar wurden 145 g Hexamethylendiamin erhalten. Die nach der Extraktion verbleibende wäßrige Natriumadipat-Lösung wurde eingedampft bis zu einer Konzentration von 27,2 Gew.-% Natriumadipat, wobei Reste des Extraktionsmittels mit entfernt wurden. Die weitere Aufarbeitung erfolgte analog Beispiel 1.

### Beispiel 3

### Diskontinuierliche Elektrolyse in einer Dreikammerelektrolysezelle gemäß Variante e)

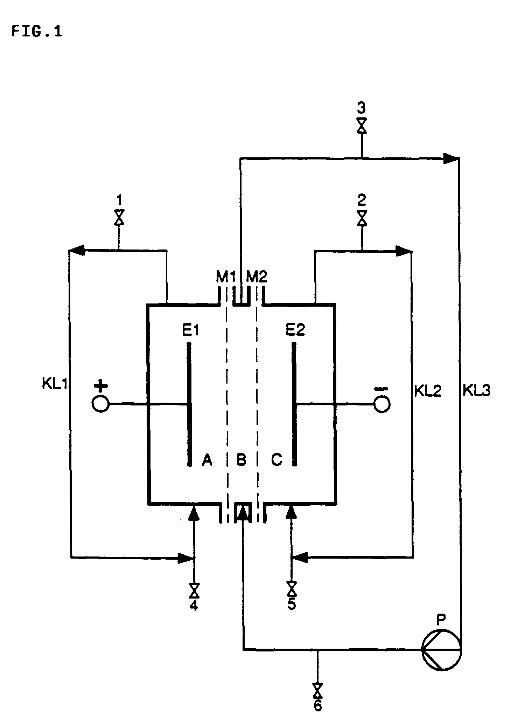

Verwendet wurde die in Figur 1 schematisch gezeigte Dreikammerelektrolysezelle mit drei Flüssigkeitskreisläufen (KL1 bis KL3). Alle produktberührenden Teile, mit Ausnahme der Elektroden, bestanden aus Polypropylen, Glas oder Quarz. Als Anode (E1) (in Kammer (A)) wurde eine Titanstreckmetallanode mit einer Fläche von 100 cm² mit einem für Sauerstoffentwicklung geeigneten Coating eingesetzt. Die Katode (E2) (in Kammer (C)) hatte ebenfalls eine Fläche von 100 cm². Sie bestand aus einem Chrom-Nickel-Edelstahl (1,4571), der mit einem für die Wasserstoffentwicklung aktivierten Nickelnetz überzogen war. Die beiden Membranen (M1 und M2) vom Typ Nafion® 324 lagen direkt auf den Elektroden (E1 bzw. E2) auf und waren durch eine 1 mm breite Mittelkammer (B) mit einem Polypropylen-Spacer voneinander getrennt.

Die Anoden- (KL1) und Katoden-(KL2)-Kreisläufe wurden aufgrund der Gasentwicklungen an den Elektroden in Naturumlauf gehalten. Der Kreislauf der Mittelkammer (B), (KL3), wurde mit einer Kreiselpumpe (P) umgewälzt. Die Strömungsgeschwindigkeit in der Mittelkammer (B) betrug 0,1 m/sec.

Eingesetzt wurden als Anolyt an der Stelle (1) 1131 g einer 5 gew.-%igen Schwefelsäure, als Katolyt an der Stelle (2) 1219 g einer 10 gew.-%igen Natronlauge, und als Mittelkammerelektrolyt an der Stelle (3) 911 g der aus Beispiel 1 erhaltenen 27 gew.-%igen Natriumadipatlösung, der 19 g einer 96 gew.-%igen Schwefelsäure zugesetzt wurden, so daß 930 g einer Lösung, enthaltend 21,7 Gew.-% Natriumadipat, 2,9 Gew.-% Adipinsäure und 2,8 Gew.-% Natriumsulfat, eingesetzt wurden.

Bei einer Temperatur von 80°C, Atmosphärendruck, einer Stromdichte von 3,0 kA/m², einer Zellspannung von 4,0 V (zu Beginn) und 5,1 V (am Ende des Versuches) wurden bei einer Stromausbeute von 78% nach einer Reaktionszeit von 2h 13min folgende Elektrolyte erhalten:

Anolyt (entnommen an Stelle (4)):
771 g einer 6,6 gew.-%igen Schwefelsäure,

Katolyt (entnommen an Stelle (5)): 1348 g einer 14,1 gew.-%igen Natronlauge,
Mittelkammerelektrolyt (entnommen an Stelle (6)):
838 g einer Lösung, enthaltend 18,7 Gew-% Adipinsäure, 2,3 Gew.-% Natriumadipat und 3,2 Gew.-% Natriumsulfat.

### Beispiel 4

### Diskontinuierliche Elektrolyse in einer Vierkammerelektrolysezelle gemäß Variante f)

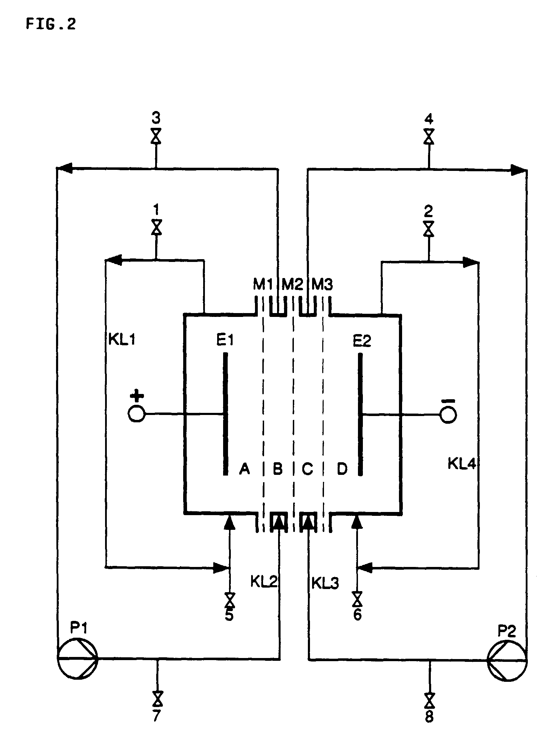

Verwendet wurde die in Figur 2 schematisch gezeigte Vierkammerelektrolysezelle mit vier Flüssigkeitskreisläufen (KL1 bis KL4). Alle produktberührenden Teile, mit Ausnahme der Elektroden, bestanden aus Polypropylen, Glas oder Quarz. Als Anode (E1) (in Kammer (A)) wurde eine Titanstreckmetallanode mit einer Fläche von 100 cm² mit einem für Sauerstoffentwicklung geeigneten Coating eingesetzt. Die Katode (E2) (in Kammer (D)) hatte ebenfalls eine Fläche von 100 cm². Sie bestand aus einem Chrom-Nickel-Edelstahl (1,4571), der mit einem für die Wasserstoffentwicklung aktivierten Nickelnetz überzogen war. Die beiden elektrodennahen Kationenaustauscher-Membranen (M1 und M3) vom Typ Nafion® 324 lagen direkt auf den Elektroden (E1 bzw. E2) auf und waren durch zwei jeweils 1 mm breite Mittelkammern, (B) und (C), mit einer in der Mitte angeordneten Anionenaustauschermembran (M2) vom Typ Tokuyama Soda® AMH getrennt. Die Mittelkammern, (B) und (C), wurden mit zwei Polypropylen-Spacern versehen, die dazu dienten, den Strömungskanal freizuhalten und den direkten Kontakt der Membranen zu verhindern.

Die Anoden- (KL1) und Katoden-(KL4)-Kreisläufe wurden aufgrund der Gasentwicklungen an den Elektroden in Naturumlauf gehalten. Die Kreisläufe der Mittelkammern (B) und (C), (KL2) und (KL3), wurden mit den Kreiselpumpen (P1) und (P2) umgewälzt. Die Strömungsgeschwindigkeiten in den Mittelkammern (B) und (C) betrugen jeweils 0,1 m/sec.

Eingesetzt wurden als Anolyt an der Stelle (1)
1099 g einer 5,1 gew.-%igen Schwefelsäure,
als Katolyt an der Stelle (2)
1103 g einer 4,1 gew.-%igen Natronlauge, und
als Elektrolyt der anodennahen Mittelkammer (B) an der Stelle (3) 1095 g einer 2,0 gew.-%igen Schwefelsäure,
als Elektrolyt der katodennahen Mittelkammer (C) an der Stelle (4) 1499 g der aus Beispiel 2 erhaltenen 27,2 gew.-%igen Natriumadipatlösung.

Während der Reaktion wurden insgesamt noch 915 g Wasser in die katodennahe Mittelkammer (C) gegeben.

Bei einer Temperatur von 80°C, Atmosphärendruck, einer Stromdichte von 3,0 kA/m², einer Zellspannung von 7,0 V (zu Beginn) und 8,7 V (am Ende des Versuches) wurden bei einer Stromausbeute von 72 % nach einer Reaktionszeit von 5h, wobei der pH-Wert in der katodennahen Mittelkammer (C) im Bereich von 10 bis 12 lag, folgende Elektrolyte erhalten:
Anolyt (entnommen an Stelle (5)) :
784 g einer 7,1 gew.-%igen Schwefelsäure,
Katolyt (entnommen an Stelle (6)):
1579 g einer 13,1 gew.-%igen Natronlauge,
Produkt der anodennahen Mittelkammer (B) (entnommen an Stelle (7)) :
2020 g einer Lösung, enthaltend 14,6 Gew-% Adipinsäure, 1,1 Gew.-% Schwefelsäure,
Produkt der katodennahen Mittelkammer (C) (entnommen an Stelle (8)):
1086 g einer 2,3 gew.-%igen Natriumadipatlösung.

## Patentansprüche

1. Verfahren zur gleichzeitigen Herstellung von Dicarbonsäuren und Diaminen aus
(a) Polymerisaten auf der Basis von Polyamiden aus Dicarbonsäuren oder deren Derivaten mit Diaminen, oder
(b) Massen, enthaltend im wesentlichen solche Polymerisate,
durch Spaltung dieser Polymerisate in ihre monomeren Bestandteile mit einer Base in wäßrigem Milieu und Abtrennung der bei der Spaltung erhaltenen Diamine, dadurch gekennzeichnet, daß man die Base in einer Mischung aus 100 bis 75 Gew.-% Wasser und 0 bis 25 Gew.-% eins C₁-C₄-Alkanols löst und die bei der Spaltung erhaltenen Dicarbonsäuresalze der Adipinsäure oder Sebazinsäure in die entsprechenden Dicarbonsäuren und Basen elektrochemisch überführt.

2. Verfahren gemäß Anspruch 1, dadurch gekennzeichnet, daß man die elektrochemische Überführung bei einer Temperatur im Bereich von 65 bis 90°C in einer drei- oder viergeteilten Membranelektrolysezelle vornimmt.

3. Verfahren gemäß Anspruch 1, dadurch gekennzeichnet, daß man die elektrochemische Überführung in einer Elektrodialyseeinheit, die aus drei Kammern besteht, bei einer Temperatur im Bereich von 40 bis 110°C vornimmt.

4. Verfahren gemäß den Ansprüchen 1 bis 3, dadurch gekennzeichnet, daß man die Adipinsäure oder Sebacinsäure aus ihren bei der elektrochemischen Behandlung erhaltenen Lösungen auskristallisiert.

5. Verfahren gemäß den Ansprüchen 1 bis 4, dadurch gekennzeichnet, daß man die bei der elektrochemischen Behandlung gewonnene Base zur Spaltung der Polymerisate in ihre monomeren Bestandteile einsetzt.

6. Verwendung der Verfahren gemäß den Ansprüchen 1 bis 5 zur Rückspaltung von Polyamiden in ihre monomeren Bestandteile.

## Claims

1. A process for coproduction of dicarboxylic acids and diamines from
(a) polymers based on polyamides of dicarboxylic acids or their derivatives with diamines, or
(b) materials comprising essentially such polymers,
by cracking these polymers into their monomeric constituents using a base in an aqueous medium and removing the resulting diamines, characterized in that the base is dissolved in a mixture of from 100 to 75% by weight of water and from 0 to 25% by weight of a C₁-C₄-alkanol and the resulting dicarboxylic acid salts of adipic acid or sebacic acid are converted into the corresponding dicarboxylic acids and bases by electrochemical means.

2. A process as claimed in claim 1, characterized in that the electrochemical conversion is carried out in a three- or four-part membrane electrolysis cell at a temperature within the range from 65 to 90°C.

3. A process as claimed in claim 1, characterized in that the electrochemical conversion is carried out in a three-compartment electrodialysis unit at a temperature within the range from 40 to 110°C.

4. A process as claimed in any of claims 1 to 3, characterized in that the adipic acid or sebacic acid is crystallized out from its solutions resulting from the electrochemical treatment.

5. A process as claimed in any of claims 1 to 4, characterized in that the base obtained from the electrochemical treatment is used for cracking the polymers into their monomeric constituents.

6. The use of the process of any of claims 1 to 5 for regenerative cracking of polyamides into their monomeric constituents.

## Revendications

1. Procédé de préparation simultanée d'acides dicarboxyliques et de diamines à partir
(a) de polymères à base de polyamides obtenus au départ d'acides dicarboxyliques ou de leurs dérivés avec des diamines, ou
(b) de masses, contenant essentiellement de tels polymères,
par la scission de ces polymères en leurs constituants monomériques avec une base, en milieu aqueux et séparation des diamines obtenues au cours de la scission, caractérisé en ce que l'on dissout la base dans un mélange de 100 à 75% en poids d'eau et de 0 à 25% en poids d'un alcanol en C₁ à C₄ et on convertit par voie électrochimique les sels d'acides dicarboxyliques de l'acide adipique ou de l'acide sébacique obtenu au cours de la scission en les acides dicarboxyliques correspondants.

2. Procédé suivant la revendication 1, caractérisé en ce que l'on entreprend la conversion électrochimique à une température qui varie de 65 à 90°C dans une cellule d'électrolyse à membrane subdivisée en trois ou en quatre.

3. Procédé suivant la revendication 1, caractérisé en ce que l'on entreprend la conversion électrochimique dans une unité d'électrodialyse qui se compose de trois chambres, à une température de 40 à 110°C.

4. Procédé suivant l'une quelconque des revendications 1 à 3, caractérisé en ce que l'on sépare l'acide adipique ou l'acide sébacique de leurs solutions obtenues au cours du traitement électrochimique par cristallisation.

5. Procédé suivant l'une quelconque des revendications 1 à 4, caractérisé en ce que l'on utilise la base obtenue au cours du traitement électrochimique pour la scission des polymères en leurs constituants monomériques.

6. Utilisation du procédé suivant l'une quelconque des revendications 1 à 5, pour la rescission de polyamides en leurs constituants monomériques.
